# EUROPÄISCHE PATENTSCHRIFT

(11) **EP 2 652 114 B1**
(45) Veröffentlichungstag und Bekanntmachung des Hinweises auf die Patenterteilung: **19.07.2017**
(21) Anmeldenummer: 11793816.7
(22) Anmeldetag: 13.12.2011
(51) Int. Cl.: C11D 3/386, C12N 9/54, C12N 9/42

(54) **LAGERSTABILES FLÜSSIGES WASCH- ODER REINIGUNGSMITTEL ENTHALTEND PROTEASE UND CELLULASE**
STORAGE-STABLE LIQUID WASHING OR CLEANING AGENT CONTAINING PROTEASE AND CELLULASE
DÉTERGENT OU PRODUIT DE NETTOYAGE LIQUIDE STABLE AU STOCKAGE CONTENANT UNE PROTÉASE ET UNE CELLULASE

(30) Priorität: 17.12.2010 DE 102010063457
(43) Veröffentlichungstag der Anmeldung: 23.10.2013
(73) Patentinhaber: BASF SE, 67056 Ludwigshafen am Rhein (DE)
(72) Erfinder: WIELAND, Susanne, 41541 Zons/Dormagen (DE); MAURER, Karl-Heinz, 40699 Erkrath (DE); O'CONNELL, Timothy, 40547 Düsseldorf (DE); HELLMUTH, Hendrik, 40237 Düsseldorf (DE)
(74) Vertreter: BASF IP Association
(86) Internationale Anmeldenummer: PCT/EP2011/072509
(87) Internationale Veröffentlichungsnummer: WO 2012/080201

(56) Entgegenhaltungen:
- WO-A1-95/23221
- WO-A1-2011/032988
- WO-A2-2014/030097
- US-B1- 6 599 730
- HERINGA JAAP ET AL: "Increasing thermal stability of subtilisin from mutations suggested by strongly interacting side-chain clusters", PROTEIN ENGINEERING, OXFORD UNIVERSITY PRESS, SURREY, GB, vol. 8, no. 1, 1 January 1995 (1995-01-01) , pages 21-30, XP009174522, ISSN: 0269-2139, DOI: 10.1093/PROTEIN/8.1.21

### SEQUENCE LISTING

<110> Henkel AG & Co. KGaA
<120> Lagerstabiles flüssiges Wasch- oder Reinigungsmittel enthaltend Protease und Cellulase
<130> H 09326 (PT018971)
<150> DE 102010063457.3
   <151> 2010-12-17
<160> 8
<170> PatentIn version 3.3
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 2
   <211> 269
   <212> PRT
   <213> Bacillus lentus
<400> 2
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Glu Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 3
   <211> 269
   <212> PRT
   <213> Bacillus lentus
<400> 3
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Asp Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 4
   <211> 269
   <212> PRT
   <213> Bacillus lentus
<400> 4
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Asn Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 5
   <211> 269
   <212> PRT
   <213> Bacillus lentus
<400> 5
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Gln Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 6
   <211> 269
   <212> PRT
   <213> Bacillus lentus
<400> 6
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Ala Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 7
   <211> 269
   <212> PRT
   <213> Bacillus lentus
<400> 7
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Gly Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 8
   <211> 269
   <212> PRT
   <213> Bacillus lentus
<400> 8
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Ser Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265

## Beschreibung

Die Erfindung liegt auf dem Gebiet der flüssigen Wasch- und Reinigungsmittel. Die Erfindung betrifft insbesondere flüssige enzymhaltige Wasch- und Reinigungsmittel, die definierte Proteasen in Kombination mit einer Cellulase enthalten, und schlägt ferner Verfahren vor, in denen solche Mittel angewendet werden. Die Erfindung betrifft ferner Verwendungen definierter Proteasen in flüssen Wasch- oder Reinigungsmitteln, die eine Cellulase enthalten.

Für Wasch- und Reinigungsmittel werden bevorzugt Proteasen vom Subtilisin-Typ eingesetzt. Die in den aus dem Stand der Technik bekannten Wasch- oder Reinigungsmitteln eingesetzten Proteasen stammen entweder ursprünglich aus Mikroorganismen, beispielsweise der Gattungen Bacillus, Streptomyces, Humicola, oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Pilze.

Insbesondere in modernen Flüssigwaschmitteln sind zunehmend weitere Enzyme enthalten, hierunter insbesondere Cellulasen (Endoglucanasen). Eine Cellulase ist ein Enzym, das die Hydrolyse von 1,4-ß-D-glucosidischen Bindungen in Cellulose (Cellobiose), und/oder Lichenin und/oder ß-D-Glucanen katalysiert. Oftmals sind sie in der Lage, die 1,4-Bindungen auch in ß-D-Glucanen zu hydrolysieren, die neben 1,4-Bindungen auch 1,3-Bindungen aufweisen. Innerhalb der EC-Klassifikation der Enzyme, dem numerischen Klassifikationssystem für Enzyme, weisen Cellulasen die EC-Nummer (engl. "Enzyme Commission number") 3.2.1.4 auf und zählen folglich zur dritten der sechs Enzymhauptklassen, den Hydrolasen (E.C.3.-.-.-), hierunter zu den Glycosylasen (E.C. 3.2.-.-) und wiederum hierunter zu den Glycosidasen (E.C. 3.2.1.-), d.h. Enzymen, die O- und/oder S-Glycosyl-Verbindungen hydrolysieren. Cellulasen sind in der Lage, Cellulose zu β-Glucose abzubauen. Cellulasen wirken folglich insbesondere gegen Rückstände in der Wäsche, die Cellulose oder Cellulosederivate enthalten, und katalysieren deren Hydrolyse.

In der internationalen Patentanmeldung WO 95/23221 sind Proteasen bzw. Protease-Varianten vom Subtilisin-Typ aus Bacillus lentus DSM 5483 offenbart, die für den Einsatz in Wasch- oder Reinigungsmitteln geeignet sind. Unter diesen Proteasen ist auch eine, die einen Aminosäureaustausch R99E, A, S oder G aufweisen kann. Ferner ist offenbart, dass die Waschmittel weitere Enzyme enthalten können, hierunter auch eine Cellulase. Die Waschmittel können fest oder flüssig sein. Jedoch geht aus dieser Schrift nicht unmittelbar und eindeutig ein flüssiges Waschmittel hervor, das eine Cellulase in Kombination mit einer Protease, die an Position 99 die Aminosäure Glutaminsäure (E) oder Asparaginsäure (D) oder die Aminosäure Asparagin (N) oder Glutamin (Q) oder die Aminosäure Alanin (A) oder Glycin (G) oder Serin (S) aufweist, enthält. Entsprechendes gilt für die europäische Patentanmeldung EP 1921147.

Ein Nachteil von protease- und cellulasehaltigen flüssigen Wasch- und Reinigungsmitteln aus dem Stand der Technik ist, dass sie nicht ausreichend lagerstabil sind und sie demnach bereits nach kurzer Zeit ein erhebliches Maß an cellulolytischer und/oder proteolytischer, insbesondere an cellulolytischer, Aktivität einbüßen. Häufig führt die Anwesenheit von Protease zum Verlust cellulolytischer Aktivität, da die Protease die Cellulase inaktiviert. Das Wasch- oder Reinigungsmittel zeigt dann keine optimale Reinigungsleistung mehr.

Der vorliegenden Erfindung liegt die Aufgabe zugrunde, den genannten Nachteil zu überwinden und protease- und cellulasehaltige flüssige Wasch- oder Reinigungsmittel bereitzustellen, die ausreichend bzw. verbessert lagerstabil sind, insbesondere hinsichtlich ihrer cellulolytischen Aktivität.

Ein Gegenstand der Erfindung ist daher ein flüssiges Wasch- oder Reinigungsmittel umfassend
(a) eine Protease, die ausgewählt ist aus der Gruppe bestehend aus
   a. Protease umfassend eine Aminosäuresequenz gemäß SEQ ID NO. 2;
   b. Protease, die eine gegenüber SEQ ID NO. 2 in mindestens einer Position veränderte Aminosäuresequenz umfasst, wobei die Veränderung in der Zählung gemäß SEQ ID NO. 1 ausgewählt ist aus der Gruppe bestehend aus:
      i. Threonin an Position 3 (3T),
      ii. Isoleucin an Position 4 (41),
      iii. Alanin, Threonin oder Arginin an Position 61 (61A, 61T oder 61 R),
      iv. Asparaginsäure oder Glutaminsäure an Position 154 (154D oder 154E),
      v. Prolin an Position 188 (188P),
      vi. Methionin an Position 193 (193M),
      vii. Isoleucin an Position 199 (1991),
      viii. Asparaginsäure, Glutaminsäure oder Glycin an Position 211 (211 D, 211 E oder 211 G),
      ix. Kombinationen der Aminosäuren (i) bis (viii);
(b) eine Cellulase.

Weiterhin ist hierin offenbart ein flüssiges Wasch- oder Reinigungsmittel umfassend
(a1) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, oder
(a2) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) aufweist, oder (a3) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Alanin (A) oder Glycin (G) oder Serin (S) aufweist, und
(b) eine Cellulase.

Überraschenderweise wurde festgestellt, dass ein flüssiges Wasch- oder Reinigungsmittel, welches die Kombination einer solchen Protease mit einer Cellulase enthält, vorteilhaft lagerstabil ist. Insbesondere weist es eine höhere cellulolytische Aktivität nach Lagerung auf im Vergleich mit einem Wasch- oder Reinigungsmittel, welches sich von einem erfindungsgemäßen Mittel lediglich durch die in dem jeweiligen Mittel vorhandene Protease unterschiedet, wobei in den zu vergleichenden Mitteln die Protease zu Lagerbeginn in gleicher Konzentration vorhanden ist, bezogen auf aktives Enzym. Eine im Rahmen der vorliegenden Erfindung vorgesehene Protease führt daher zu einer verminderten Inaktivierung der Cellulase. Die verminderte Inaktivierung von Cellulase durch die im Rahmen der vorliegenden Erfindung vorgesehene Protease beruht jedoch nicht auf einer unzureichenden Proteaseaktivität.

Ein erfindungsgemäßes Mittel weist diesbezüglich und vorzugsweise eine weiterhin gute, insbesondere vorteilhafte, Reinigungsleistung an protease-sensitiven Anschmutzungen auf. Eine solche Reinigungsleistung hinsichtlich mindestens einer protease-sensitiven Anschmutzung tritt insbesondere auch bei niedrigen Temperaturen, auf, beispielsweise zwischen 10°C und 50°C, bevorzugt zwischen 10°C und 40°C oder zwischen 20°C und 40°C. Ein solches Mittel ermöglicht daher eine zufrieden stellende oder verbesserte Entfernung von mindestens einer, bevorzugt von mehreren protease-sensitiven Anschmutzungen auf Textilien und/oder harten Oberflächen, beispielsweise Geschirr.

Hinsichtlich der einleitend erwähnten internationalen Patentanmeldung WO 95/23221 handelt es bei der vorliegenden Erfindung somit um eine besonders vorteilhafte Auswahl, die zum Erhalt eines leistungsfähigen und lagerstabilen Flüssigwaschmittels führt, insbesondere hinsichtlich der proteolytischen und/oder cellulolytischen Aktivität des Mittels bzw. Restaktivität des Mittels nach Lagerung.

Unter Reinigungsleistung wird im Rahmen der Erfindung die Aufhellungsleistung an einer oder mehreren Anschmutzungen, insbesondere Wäscheanschmutzungen, verstanden. Beispiele für solche Anschmutzungen sind Blut-Milch/Tusche auf Baumwolle, Voll-Ei/Pigment auf Baumwolle, Schokolade-Milch/Tusche auf Baumwolle, Erdnuss Öl-Pigment/Tusche auf Polyester/Baumwolle, Gras auf Baumwolle oder Kakao auf Baumwolle, insbesondere derart wie weiter unten angegeben. Im Rahmen der Erfindung weist sowohl das Wasch- oder Reinigungsmittel, welches die Protease und die Cellulase umfasst bzw. die durch dieses Mittel gebildete Wasch- bzw. Reinigungsflotte, als auch die Protease bzw. die Cellulase selbst eine jeweilige Reinigungsleistung auf. Die Reinigungsleistung der Enzyme trägt somit zur Reinigungsleistung des Mittels bzw. der durch das Mittel gebildeten Wasch- bzw. Reinigungsflotte bei. Die Reinigungsleistung wird bevorzugt ermittelt wie weiter unten angegeben.

Unter Wasch- bzw. Reinigungsflotte wird diejenige das Wasch- oder Reinigungsmittel enthaltende Gebrauchslösung verstanden, die auf Textilien oder Gewebe (Waschflotte) bzw. harte Oberflächen (Reinigungsflotte) einwirkt und damit mit den auf Textilien bzw. Geweben oder harten Oberflächen vorhandenen Anschmutzungen in Kontakt kommt. Üblicherweise entsteht die Wasch- bzw. Reinigungsflotte, wenn der Wasch- oder Reinigungsvorgang beginnt und das Wasch- oder Reinigungsmittel beispielsweise in einer Waschmaschine oder in einem anderen geeigneten Behältnis mit Wasser verdünnt wird.

Eine Lagerstabilität im Sinne der Erfindung liegt vor, wenn ein erfindungsgemäßes Wasch- oder Reinigungsmittel nach einer Lagerung eine höhere Cellulase-Aktivität aufweist im Vergleich zu einer Kontrollzusammensetzung, die sich nur durch die in der Kontrollzusammensetzung enthaltene Protease von dem erfindungsgemäßen Wasch- oder Reinigungsmittel unterscheidet. Nach Lagerung weist ein erfindungsgemäßes Wasch- oder Reinigungsmittel daher eine höhere Restaktivität der Cellulase auf im Vergleich zur Kontrolle. Beide zu vergleichenden Mittel weisen bei Lagerbeginn daher die gleiche Cellulasemenge bzw. -konzentration und/oder cellulolytische Ausgangsaktivität auf. Weiterhin ist in beiden Mitteln die Protease zu Lagerbeginn in gleicher Konzentration vorhanden, bezogen auf aktives Enzym, und beide Mittel werden auf die gleiche Art und Weise behandelt, insbesondere betreffend die Bedingungen der Lagerung und die Bestimmung der Enzymaktivität. Zunehmend bevorzugt erfolgt die Lagerung für mindestens 24 Stunden, 48 Stunden, 72 Stunden, 5 Tage, 1 Woche, 2 Wochen, 3 Wochen, 4 Wochen oder 8 Wochen. Weiter bevorzugt erfolgt die Lagerung bei einer Temperatur von 20°C, 25°C oder 30°C, besonders bevorzugt bei 30°C.

Die Enzymaktivität kann diesbezüglich - abgestimmt auf den jeweiligen Enzymtyp - in fachüblicher Art und Weise erfolgen. Methoden zur Aktivitätsbestimmung sind dem Fachmann auf dem Gebiet der Enzymtechnologie geläufig und werden von ihm routinemäßig angewendet. Verfahren zur Bestimmung der Proteaseaktivität sind beispielsweise offenbart in Tenside, Band 7 (1970), S. 125-132. Die proteolytische Aktivität kann ferner bestimmt werden über die Freisetzung des Chromophors para-Nitroanilin (pNA) aus dem Substrat suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6 und einer Wellenlänge von 410 nm. Die Messzeit beträgt 5 min. bei einem Messintervall von 20s bis 60s. Die Proteaseaktivität wird vorzugsweise in PE (Protease-Einheiten) angegeben.

Die Cellulaseaktivität wird in fachüblicher Weise bestimmt. Vorzugsweise wird die Cellulaseaktivität bestimmt wie nachstehend angegeben. Cellulasen setzen aus CMC (Carboxymethylcellulose) Glucose frei. Unter definierten Reaktionsbedingungen (100 mM Natriumphosphatpuffer pH 7.5, 40°C, 15 min) werden die zu untersuchenden Proben mit einem Substrat (1,25% CMC) inkubiert. Diese reagieren mit p-Hydroxybenzoesäure-hydrazid (PAHBAH) unter Anwesenheit von Bismuth zu einem gelben Farbstoff, der photometrisch bei 410 nm bestimmt werden kann. Bedingung ist ein alkalischer pH-Wert während der Farbreaktion. Die der Färbung entsprechende Menge des freigesetzten Zuckers ist dabei ein Maß für die Enzymaktivität (vgl. Lever, Anal. Biochem., 1972, 47 & 1977,81).

Besonders bevorzugt wird das Vorliegen einer Enzymstabilisierung im Sinne der vorliegenden Erfindung ermittelt wie vorstehend angegeben unter Verwendung eines Protease-und Cellulasehaltigen flüssigen Wasch- oder Reinigungsmittels, das für mindestens vier und höchstens 8 Wochen bei einer Temperatur von 30°C gelagert wird, wobei die proteolytische Aktivität bestimmt wird über die Freisetzung des Chromophors para-Nitroanilin (pNA) aus dem Substrat suc-AAPF-pNA, und die cellulolytische Aktivität bestimmt wird wie vorstehend angegeben.

Weiterhin ist hierin offenbart ein Wasch- oder Reinigungsmittel enthaltene Protease umfasst eine Aminosäuresequenz, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) oder die Aminosäure Asparagin (N) oder Glutamin (Q) oder die Aminosäure Alanin (A) oder Glycin (G) oder Serin (S) aufweist. Zunehmend bevorzugt ist die Aminosäuresequenz zu mindestens 81%, 82%, 83%, 84%, 85%, 86%, 87%, 88%, 89%, 90%, 91%, 92%, 93%, 94%, 95%, 96%, 97%, 98% und ganz besonders bevorzugt zu 99% identisch zu der in SEQ ID NO. 1 angegebenen Aminosäuresequenz. SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird.

Erfindungsgemäß hat sich gezeigt, dass durch den Zusatz einer solchen Protease zu einem flüssigen Wasch- oder Reinigungsmittel, welches eine Cellulase enthält, ein besonders lagerstabiles Flüssigwaschmittel erhalten wird, insbesondere hinsichtlich dessen verbleibender cellulolytischer Aktivität nach Lagerung, insbesondere nach einer Lagerdauer von zunehmend bevorzugt 24 Stunden, 48 Stunden, 72 Stunden, 5 Tage, 1 Woche, 2 Wochen, 3 Wochen, 4 Wochen oder 8 Wochen.

Eine in einem erfindungsgemäßen Wasch- oder Reinigungsmittel enthaltene Protease weist eine proteolytische Aktivität auf, das heißt, sie ist zur Hydrolyse von Peptidbindungen eines Polypeptids bzw. Proteins befähigt. Sie ist daher ein Enzym, welches die Hydrolyse von Peptidbindungen katalysiert und dadurch in der Lage ist, Peptide oder Proteine zu spalten. Sie ist insbesondere eine Subtilase und besonders bevorzugt ein Subtilisin.

Eine Cellulase ist ein Enzym wie einleitend beschrieben. Für Cellulasen können synonyme Begriffe verwendet werden, insbesondere Endoglucanase, Endo-1,4-beta-Glucanase, Carboxymethylcellulase, Endo-1,4-beta-D-Glucanase, beta-1,4-Glucanase, beta-1,4-Endoglucanhydrolase, Celludextrinase oder Avicelase. Entscheidend dafür, ob ein Enzym eine Cellulase im Sinne der Erfindung ist, ist deren Fähigkeit zur Hydrolyse von 1,4-ß-D-glucosidischen Bindungen in Cellulose.

Erfindungsgemäß konfektionierbare Cellulasen (Endoglucanasen, EG) umfassen beispielsweise die pilzliche, Endoglucanase(EG)-reiche Cellulase-Präparation beziehungsweise deren Weiterentwicklungen, die von dem Unternehmen Novozymes unter dem Handelsnamen Celluzyme® angeboten wird. Die ebenfalls von dem Unternehmen Novozymes erhältlichen Produkte Endolase® und Carezyme® basieren auf der 50 kD-EG, beziehungsweise der 43 kD-EG aus Humicola insolens DSM 1800. Weitere einsetzbare Handelsprodukte dieses Unternehmens sind Cellusoft®, Renozyme® und Celluclean®. Weiterhin einsetzbar sind beispielsweise Cellulasen, die von dem Unternehmen AB Enzymes, Finnland, unter den Handelsnamen Ecostone® und Biotouch® erhältlich sind, und die zumindest zum Teil auf der 20 kD-EG aus Melanocarpus basieren. Weitere Cellulasen von dem Unternehmen AB Enzymes sind Econase® und Ecopulp®. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax® erhältlich ist. Weitere verwendbare Handelsprodukte des Unternehmens Danisco/Genencor sind "Genencor detergent cellulase L" und IndiAge®Neutra. Auch durch Punktmutationen erhältliche Varianten dieser Enzyme können erfindungsgemäß eingesetzt werden. Besonders bevorzugte Cellulasen sind Thielavia terrestris Cellulasevarianten, die in der internationalen Offenlegungsschrift WO 98/12307 offenbart sind, Cellulasen aus Melanocarpus, insbesondere Melanocarpus albomyces, die in der internationalen Offenlegungsschrift WO 97/14804 offenbart sind, Cellulasen vom EGIII-Typ aus Trichoderma reesei, die in der europäischen Patentanmeldung EP 1 305 432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Patentanmeldung mit einbezogen.

In einer weiteren Ausführungsform der Erfindung ist das Wasch- oder Reinigungsmittel dadurch gekennzeichnet, dass die Protease ferner in der Zählung gemäß SEQ ID NO. 1 mindestens eine der folgenden Aminosäuren aufweist:
(a) Threonin an Position 3 (3T),
(b) Isoleucin an Position 4 (41),
(c) Alanin, Threonin oder Arginin an Position 61 (61A, 61T oder 61 R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (1991),
(h) Asparaginsäure, Glutaminsäure oder Glycin an Position 211 (211 D, 211 E oder 211 G),
(i) Kombinationen der Aminosäuren (a) bis (h).

Neben einer der genannten Aminosäuren an Position 99 weist die Protease daher eine oder mehrere der vorstehend genannten Aminosäuren an den jeweiligen Positionen auf. Diese Aminosäuren können weitere vorteilhafte Eigenschaften bewirken und/oder bereits vorhandene Eigenschaften noch verstärken. Insbesondere bewirken die vorstehend genannten Aminosäuren eine Steigerung der proteolytischen Aktivität und/oder der Stabilität der Protease in einem flüssigen Wasch- oder Reinigungsmittel bzw. in der durch dieses Wasch- oder Reinigungsmittel gebildeten Waschflotte. Durch den Zusatz einer solchen Protease zu einem flüssigen Wasch- oder Reinigungsmittel, welches eine Cellulase enthält, wird somit ebenfalls ein besonders lagerstabiles Flüssigwaschmittel erhalten, insbesondere hinsichtlich dessen verbleibender cellulolytischer Aktivität nach Lagerung, aber vorzugsweise auch hinsichtlich dessen verbleibender proteolytischer Aktivität nach Lagerung, insbesondere nach einer Lagerdauer von zunehmend bevorzugt 24 Stunden, 48 Stunden, 72 Stunden, 5 Tage, 1 Woche, 2 Wochen, 3 Wochen, 4 Wochen oder 8 Wochen. Ferner zeigt ein solches Mittel verbesserte Reinigungsleistungen an protease- und/oder cellulase-sensitiven Anschmutzungen.

Die Aminosäurepositionen werden hierbei durch ein Alignment der Aminosäuresequenz der einzusetzenden Protease mit der Aminosäuresequenz der Protease aus Bacillus lentus, wie sie in SEQ ID NO. 1 angegeben ist, definiert. Da die Protease aus Bacillus lentus im Stand der Technik ein wichtiges Referenzmolekül zur Beschreibung von Proteasen und von Aminosäureveränderungen darstellt ist es vorteilhaft, in der Zuordnung der Aminosäurepositionen auf die Zählung der Protease aus Bacillus lentus (SEQ ID NO. 1) Bezug zu nehmen. Weiterhin richtet sich die Zählung nach dem reifen (maturen) Protein. Diese Zuordnung ist insbesondere auch anzuwenden, wenn die Aminosäuresequenz der einzusetzenden Protease eine höhere Zahl von Aminosäurenresten umfasst als die Protease aus Bacillus lentus gemäß SEQ ID NO. 1. Ausgehend von den genannten Positionen in der Aminosäuresequenz der Protease aus Bacillus lentus sind die Aminosäurepositionen in einer erfindungsgemäß einzusetzenden Protease diejenigen, die eben diesen Positionen in einem Alignment zugeordnet sind.

Neben Position 99 besonders vorteilhafte Positionen sind demnach die Positionen 3, 4, 61, 154, 188, 193, 199 und 211, zuzuordnen in einem Alignment mit SEQ ID NO. 1 und damit in der Zählung gemäß SEQ ID NO. 1. In den genannten Positionen liegen in dem Wildtypmolekül der Protease aus Bacillus lentus folgende Aminosäurereste: S3, V4, G61, S154, A188, V193, V199, und L211. Besonders bevorzugt sind die Aminosäuren 3T, 41, 61A, 154D, 154E, 211D, 211G und 211 E, sofern die entsprechenden Positionen in einer erfindungsgemäß einzusetzenden Protease nicht schon von einer dieser bevorzugten Aminosäuren eingenommen werden. Die Austausche 3T und 4I führen beispielsweise über einen Stabilisierungseffekt auf das Molekül zu einer Verbesserung der Lagerstabilität und der Reinigungsleistung der Protease und damit zu einer verbesserten Reinigungsleistung eines erfindungsgemäßen flüssigen Wasch- oder Reinigungsmittels, welches die Protease enthält.

Sind eine oder mehrere der vorstehend genannten Aminosäuren an der jeweiligen Position verwirklicht, ergeben sich zusätzlich zu Position 99 weitere Sequenzabweichungen von SEQ ID NO. 1, da SEQ ID NO. 1 eine andere Aminosäure in der jeweiligen Position aufweist. In Abhängigkeit von der Anzahl der vorliegenden Sequenzabweichungen von SEQ ID NO.1 ergeben sich daher unterschiedliche maximale Identitätswerte, die eine erfindungsgemäß einzusetzende Protease zu SEQ ID NO. 1 aufweisen kann, selbst wenn sie in allen übrigen Aminosäuren mit SEQ ID NO. 1 übereinstimmen sollte. Dieser Sachverhalt ist für jede mögliche Kombination der vorgeschlagenen Aminosäuren im Einzelfall zu berücksichtigen und ist ferner auch abhängig von der Länge der Aminosäuresequenz der Protease. Beispielsweise beträgt die maximale Identität bei einer, zwei, drei, vier, fünf, sechs, sieben, acht oder neun Sequenzveränderungen 99,63%, 99,26%, 98,88%, 98,51%, 98,14%, 97,77%, 97,40%, 97,03% bzw. 96,65% bei einer 269 Aminosäuren langen Aminosäuresequenz, beziehungsweise 99,64%, 99,27%, 98,91%, 98,55%, 98,18%, 97,82%, 97,45%, 97,09% bzw. 96,73% bei einer 275 Aminosäuren langen Aminosäuresequenz.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F.,Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz. Weiterhin ist hierin offenbart, dass die Protease eine Aminosäuresequenz umfasst, die zu der in SEQ ID NO. 1 angegebenen Aminosäuresequenz wie vorstehend angegeben identisch ist und die aus einer Protease gemäß SEQ ID NO. 1 durch ein- oder mehrfache konservative Aminosäuresubstitution erhalten wird bzw. erhältlich ist, wobei die Protease an Position 99 noch immer eine der für diese Position vorgesehenen Aminosäuren aufweist wie vorstehend beschrieben. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

In einer weiteren Ausführungsform der Erfindung ist ein erfindungsgemäßes Wasch- oder Reinigungsmittel ferner dadurch gekennzeichnet, dass seine Reinigungsleistung mindestens derjenigen eines Wasch- bzw. Reinigungsmittels entspricht, das eine Protease beinhaltet, die eine Aminosäuresequenz umfasst, die der in SEQ ID NO. 2 angegebenen Aminosäuresequenz entspricht. Weiterhin ist hierin ein Wasch- oder Reinigungsmittel offenbart, ferner dadurch gekennzeichnet, dass seine Reinigungsleistung mindestens derjenigen eines Wasch- bzw. Reinigungsmittels entspricht, das eine Protease beinhaltet, die eine Aminosäuresequenz umfasst, die der in SEQ ID NO. 3 oder SEQ ID NO. 4 oder SEQ ID NO. 5 oder SEQ ID NO. 6 oder SEQ ID NO. 7 oder SEQ ID NO. 8, besonders bevorzugt der in SEQ ID NO. 2, angegebenen Aminosäuresequenz entspricht. Die Reinigungsleistung wird in einem Waschsystem bestimmt, das ein cellulasehaltiges Waschmittel in einer Dosierung zwischen 2,0 und 9,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer oder mehrerer der Anschmutzungen Blut-Milch/Tusche auf Baumwolle, Voll-Ei/Pigment (Ganzei/Ruß) auf Baumwolle, Erdnuss Öl-Pigment/Tusche auf Polyester/Baumwolle und Gras auf Baumwolle, insbesondere gegenüber einer oder mehrerer der Anschmutzungen - Blut-Milch/Tusche auf Baumwolle: Produkt Nr. C-05 erhältlich von CFT (Center For Testmaterials) B.V. Vlaardingen, Niederlande
- Voll-Ei/Pigment (Ganzei/Ruß) auf Baumwolle: Produkt Nr. 10N erhältlich von der Firma wfk Testgewebe GmbH; Brüggen-Bracht, Deutschland, oder Produkt C-S-37 erhältlich von CFT (Center For Testmaterials) B.V. Vlaardingen, Niederlande
- Erdnuss Öl-Pigment/Tusche auf Polyester/Baumwolle: Produkt Nr. PC-10 erhältlich von CFT (Center For Testmaterials) B.V. Vlaardingen, Niederlande
- Gras auf Baumwolle: Produkt Nr. 164 erhältlich von der Firma Eidgenössische Material- und Prüfanstalt (EMPA) Testmaterialien AG, St. Gallen, Schweiz,
bestimmt wird durch Messung des Weißheitsgrades der gewaschenen Textilien, der Waschvorgang für mindestens 30 Minuten, optional 60 Minuten, bei einer Temperatur von 20°C erfolgt und das Wasser eine Wasserhärte zwischen 15,5 und 16,5° (deutsche Härte) aufweist.

Das Waschmittel für das Waschsystem ist ein flüssiges Waschmittel, das wie folgt zusammengesetzt (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1% Borsäure, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001% Farbstoff, 0,0002-0,06% Cellulase (aktives Protein), vorzugsweise Ecostone N400® (Cellulasepräparation der Firma AB Enzymes), Rest demineralisiertes Wasser. Die Protease wird in dem Waschmittel in einer Konzentration von 0,001-0,1%, vorzugsweise von 0,01 bis 0,06%, eingesetzt, bezogen auf aktives Protein. Die Dosierung des flüssigen Waschmittels beträgt zwischen 2,0 und 9,0, vorzugsweise zwischen 3,0 und 8,2, zwischen 4,0 und 7,5 und besonders bevorzugt 4,7 Gramm pro Liter Waschflotte. Gewaschen wird in einem pH-Wertebereich zwischen pH 8 und pH 10,5, bevorzugt zwischen pH 8 und pH 9. Weder die Protease- noch die Cellulaseaktivität in der Waschflotte sind zu Waschbeginn gleich Null.

Der Weißheitsgrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

In einer weiteren Ausführungsform der Erfindung ist ein erfindungsgemäßes Wasch- oder Reinigungsmittel ferner dadurch gekennzeichnet, dass seine Lagerstabilität mindestens derjenigen eines Wasch- bzw. Reinigungsmittels entspricht, das eine Protease beinhaltet, die eine Aminosäuresequenz umfasst, die der in SEQ ID NO. 2 angegebenen Aminosäuresequenz entspricht. Weiterhin ist hierin ein Wasch- oder Reinigungsmittel offenbart, ferner dadurch gekennzeichnet, dass seine Lagerstabilität mindestens derjenigen eines Wasch- bzw.

Reinigungsmittels entspricht, das eine Protease beinhaltet, die eine Aminosäuresequenz umfasst, die der in SEQ ID NO. 2 oder SEQ ID NO. 3 oder SEQ ID NO. 4 oder SEQ ID NO. 5 oder SEQ ID NO. 6 oder SEQ ID NO. 7 oder SEQ ID NO. 8, besonders bevorzugt der in SEQ ID NO. 2, angegebenen Aminosäuresequenz entspricht. Eine solche Lagerstabilität liegt dann vor, wenn das erfindungsgemäße Wasch- oder Reinigungsmittel nach einer Lagerung von acht Wochen bei 30°C eine gleiche oder höhere Cellulase-Aktivität aufweist als das zum Vergleich heranzuziehende Wasch- oder Reinigungsmittel, wobei sich das erfindungsgemäße Mittel nur durch die enthaltene Protease von dem als Vergleich heranzuziehenden Wasch- oder Reinigungsmittel unterscheidet.

Besonders bevorzugt handelt es sich bei dem zum Vergleich heranzuziehenden Mittel um ein flüssiges Waschmittel, das wie folgt zusammengesetzt (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1% Borsäure, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001 % Farbstoff, 0,0002-0,06% Cellulase (aktives Protein), vorzugsweise Ecostone N400® (Cellulasepräparation der Firma AB Enzymes), Rest demineralisiertes Wasser. Die Protease wird in dem Waschmittel in einer Konzentration von 0,001-0,1%, vorzugsweise von 0,01 bis 0,06%, eingesetzt, bezogen auf aktives Protein.

Bei Lagerbeginn weisen beide zu vergleichenden Mittel die gleiche cellulolytische Ausgangsaktivität auf, enthalten die Protease in gleicher Konzentration bezogen auf aktives Enzym, und beide Mittel werden auf die gleiche Art und Weise behandelt. Die proteolytische Aktivität in den Mitteln wird jeweils bestimmt über die Freisetzung des Chromophors para-Nitroanilin (pNA) aus dem Substrat suc-AAPF-pNA, und deren cellulolytische Aktivität wird jeweils bestimmt wie vorstehend angegeben. Die Ausgangsaktivitäten für die Protease und die Cellulase in dem jeweiligen Mittel sind nicht gleich Null.

Durch den jeweils aktivitätsgleichen Einsatz der Cellulase und den konzentrationsgleichen Einsatz der Proteasen, bezogen auf aktives Protein, wird sichergestellt, dass auch bei einem etwaigen Auseinanderklaffen des Verhältnisses von Aktivsubstanz zu Gesamtprotein (die Werte der spezifischen Aktivität) die real vorliegenden enzymatischen Eigenschaften verglichen werden.

Soweit nicht anders angegeben wird im Rahmen der vorliegenden Erfindung jeweils auf das Gewicht des flüssigen Waschmittels Bezug genommen, d.h. die Angaben sind bezogen auf dessen Gewicht.

Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen Bedigungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

Ferner kann auch die reife Protease an ihrem N-terminalen und/oder C-terminalen Ende verkürzt sein, so dass eine gegenüber SEQ ID NO. 1 oder SEQ ID NO. 2 oder SEQ ID NO. 3 oder SEQ ID NO. 4 oder SEQ ID NO. 5 oder SEQ ID NO. 6 oder SEQ ID NO. 7 oder SEQ ID NO. 8 verkürzte Protease, also ein Fragment, in dem hierin offenbarten Wasch- oder Reinigungsmittel enthalten ist. Alle Identitätsangaben beziehen sich in diesem Fall auf denjenigen Bereich, in dem das jeweilige Fragment in einem Alignment SEQ ID NO. 1 zugeordnet ist. Das jeweilige Fragment beinhaltet aber in jedem Fall diejenige Position, die der Position 99 in einem Alignment mit SEQ ID NO. 1 zugeordnet ist, und weist an dieser Position eine entsprechende Aminosäure auf. Vorteilhafterweise beinhaltet es auch eine oder mehrere der weiteren vorstehend beschriebenen Positionen und weist dort eine entsprechende Aminosäure auf. Ferner ist ein solches Fragment proteolytisch aktiv. Ein diesbezüglich weiter bevorzugtes Fragment umfasst eine Aminosäuresequenz, die über eine Länge von mindestens 50 oder mindestens 60, 70, 80, 90, 100, 110, 120, 130, 140, 150, 160, 170, 180, 190, 200, 210, 220, 230, 240, 250, 260, 265, 266, 267 oder 268 zusammenhängenden Aminosäurepositionen mit SEQ ID NO. 1 oder SEQ ID NO. 2 oder SEQ ID NO. 3 oder SEQ ID NO. 4 oder SEQ ID NO. 5 oder SEQ ID NO. 6 oder SEQ ID NO. 7 oder SEQ ID NO. 8 übereinstimmt unter Berücksichtigung der vorstehend genannten Aminosäuren für die Position 99 und optional ferner für die Positionen 3 und/oder 4 und/oder 61 und/oder 154 und/oder 188 und/oder 193 und/oder 199 und/oder 211. Besonders bevorzugt entspricht die Reinigungsleistung und/oder Lagerstabilität eines flüssigen Wasch- oder Reinigungsmittels mit einem solchen Fragment mindestens derjenigen eines Wasch- bzw. Reinigungsmittels, das eine Protease beinhaltet, die eine Aminosäuresequenz umfasst, die der in SEQ ID NO. 2 angegebenen Aminosäuresequenz entspricht, jeweils bestimmt wie vorstehend angegeben. Weiterhin entspricht die Reinigungsleistung und/oder Lagerstabilität eines hierein offenbarten flüssigen Wasch- oder Reinigungsmittels mit einem solchen Fragment mindestens derjenigen eines Wasch- bzw. Reinigungsmittels, das eine Protease beinhaltet, die eine Aminosäuresequenz umfasst, die der in SEQ ID NO. 3 oder SEQ ID NO. 4 oder SEQ ID NO. 5 oder SEQ ID NO. 6 oder SEQ ID NO. 7 oder SEQ ID NO. 8 angegebenen Aminosäuresequenz entspricht, jeweils bestimmt wie vorstehend angegeben. Ein weiterer Gegenstand der Erfindung ist ein flüssiges Wasch- oder Reinigungsmittel umfassend
(a) eine Protease, die ausgewählt ist aus der Gruppe bestehend aus
   a. Protease umfassend eine Aminosäuresequenz gemäß SEQ ID NO. 2;
   b. Protease, die eine gegenüber SEQ ID NO. 2 in mindestens einer Position veränderte Aminosäuresequenz umfasst, wobei die Veränderung in der Zählung gemäß SEQ ID NO. 1 ausgewählt ist aus der Gruppe bestehend aus:
      i. Threonin an Position 3 (3T),
      ii. Isoleucin an Position 4 (41),
      iii. Alanin, Threonin oder Arginin an Position 61 (61A, 61T oder 61 R),
      iv. Asparaginsäure oder Glutaminsäure an Position 154 (154D oder 154E),
      v. Prolin an Position 188 (188P),
      vi. Methionin an Position 193 (193M),
      vii. Isoleucin an Position 199 (1991),
      viii.Asparaginsäure, Glutaminsäure oder Glycin an Position 211 (211 D, 211 E oder 211 G),
      ix. Kombinationen der Aminosäuren (i) bis (viii);
(b) eine Cellulase.

Offenbart hierin ist weiterhin ein flüssiges Wasch- oder Reinigungsmittel umfassend
(a) eine Protease, die ausgewählt ist aus der Gruppe bestehend aus
   a. Protease umfassend eine Aminosäuresequenz gemäß SEQ ID NO. 3 oder SEQ ID NO. 4 oder SEQ ID NO. 5 oder SEQ ID NO. 6 oder SEQ ID NO. 7 oder SEQ ID NO. 8;
   b. Protease, die eine gegenüber SEQ ID NO. 3 oder SEQ ID NO. 4 oder SEQ ID NO. 5 oder SEQ ID NO. 6 oder SEQ ID NO. 7 oder SEQ ID NO. 8 in mindestens einer Position veränderte Aminosäuresequenz umfasst, wobei die Veränderung in der Zählung gemäß SEQ ID NO. 1 ausgewählt ist aus der Gruppe bestehend aus:
      i. Threonin an Position 3 (3T),
      ii. Isoleucin an Position 4 (4I),
      iii. Alanin, Threonin oder Arginin an Position 61 (61A, 61T oder 61 R),
      iv. Asparaginsäure oder Glutaminsäure an Position 154 (154D oder 154E),
      v. Prolin an Position 188 (188P),
      vi. Methionin an Position 193 (193M),
      vii. Isoleucin an Position 199 (199I),
      viii.Asparaginsäure, Glutaminsäure oder Glycin an Position 211 (211 D, 211 E oder 211 G),
      ix. Kombinationen der Aminosäuren (i) bis (viii);
(b) eine Cellulase.

Diese Proteasen werden ganz besonders bevorzugt in einem erfindungsgemäßen flüssigen Wasch- oder Reinigungsmittel eingesetzt. Sie werden ausgehend von SEQ ID NO. 1 erhalten durch die Substitution der Aminosäure Arginin an Position 99 durch die Aminosäure Glutaminsäure (E) in der Zählung gemäß SEQ ID NO. 1. Diese Aminosäuresequenz ist im Sequenzprotokoll als SEQ ID NO. 2 angegeben. Die weiterhin hierin offenbarten Proteasen werden ausgehend von SEQ ID NO. 1 erhalten durch die Substitution der Aminosäure Arginin an Position 99 durch die Aminosäure Asparaginsäure (D) oder die Aminosäure Asparagin (N) oder Glutamin (Q) oder die Aminosäure Alanin (A) oder Glycin (G) oder Serin (S) in der Zählung gemäß SEQ ID NO. 1. Diese Aminosäuresequenzen sind im Sequenzprotokoll als SEQ ID NO. 3, SEQ ID NO. 4, SEQ ID NO. 5, SEQ ID NO. 6, SEQ ID NO. 7 und SEQ ID NO. 8 angegeben. Ferner können diese Proteasen zusätzlich zu der für Position 99 vorgesehenen Aminosäure eine oder mehrere der vorstehend erläuterten Aminosäuren in den Positionen 3, 4, 61, 154, 188, 193, 199 und 211 aufweisen, zuzuordnen in einem Alignment mit SEQ ID NO. 1 und damit in der Zählung gemäß SEQ ID NO. 1. Die genannten Aminosäuren für diese Positionen bewirken auch bei diesen Proteasen weitere vorteilhafte Eigenschaften und/oder verstärken bereits vorhandene Eigenschaften noch. Insbesondere bewirken sie eine Steigerung der proteolytischen Aktivität und/oder der Stabilität der Protease in einem flüssigen Wasch- oder Reinigungsmittel bzw. in der durch dieses Wasch- oder Reinigungsmittel gebildeten Waschflotte. Alle vorstehenden Ausführungen - sofern anwendbar - gelten für diese besonders bevorzugten Proteasen entsprechend.

Ein erfindungsgemäßes Mittel enthält die Protease zunehmend bevorzugt in einer Menge von 1 x 10⁻⁸-5 Gew.-%, von 0,0001-1 Gew.-%, von 0,0005-0,5 Gew.-%, von 0,001 bis 0,1 Gew.-% und besonders bevorzugt von 0,001 bis 0,06 Gew.-%, bezogen auf aktives Protein. Ein erfindungsgemäßes Mittel enthält die Cellulase zunehmend bevorzugt in einer Menge von 1 x 10⁻⁸-5 Gew.-%, von 0,00001-1 Gew.-%, von 0,00005-0,5 Gew.-%, von 0,0001 bis 0,1 Gew.-% und besonders bevorzugt von 0,0001 bis 0,065 Gew.-%, bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration erfolgte diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die Protease und/oder die Cellulase können ferner an Trägerstoffen adsorbiert und/oder in Hüllsubstanzen eingebettet sein, um sie gegen vorzeitige Inaktivierung zu schützen. In der Waschflotte, also unter Anwendungsbedingungen, wird das Enzym dann freigesetzt und kann seine katalytische Wirkung entfalten.

In einer weiteren Ausführungsform der Erfindung ist das Wasch- oder Reinigungsmittel dadurch gekennzeichnet, dass es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

Es wurde festgestellt, dass der Zusatz solcher Substanzen die Reinigungsleistung von Wasch- und Reinigungsmitteln, insbesondere von flüssigen Wasch- oder Reinigungsmitteln, welche Proteasen und Cellulasen enthalten, insbesondere solche wie vorstehend beschrieben, weiter verbessert, insbesondere bei vergleichsweise niedrigen Temperaturen, insbesondere zwischen 10°C und 50°C, zwischen 10°C und 40°C, zwischen 10°C und 30°C und/oder zwischen 20°C und 40°C. Insbesondere in Kombination mit einer erfindungsgemäß einzusetzenden Protease tritt eine synergistische Wirkung auf, vor allem hinsichtlich der Entfernung von mindestens einer protease-sensitiven Anschmutzung, insbesondere einer solchen wie vorstehend angegeben.

Bei den vorstehend unter i. angegebenen Substanzen handelt es sich um anionische oder polyanionische Substanzen, d.h. diese Substanzen tragen mindestens eine und bevorzugt mehrere negative Ladungen. Bevorzugt handelt es sich um ein Polymer mit mindestens einem negativ gelandenen Monomer, bevorzugt mit mehreren negativ geladenen Monomeren. Erfindungsgemäß bevorzugt ist dieses Polymer daher ein negativ geladenes Polymer. Bevorzugt sind beispielsweise Polymere organischer Säuren bzw. deren Salze, insbesondere Polyacrylate und/oder Poly-Zuckersäuren und/oder Polyarcylat-copolymere und/oder Poly-zucker-copolymere. Diesbezüglich weitere bevorzugte Verbindungen sind Polyacrylsulfonate oder Polycarboxylate und deren Salze, Copolymere oder Salze der Coploymere.

Beispiele für besonders bevorzugt einzusetzende Substanzen sind Acusol 587D (Polyacrylsulfonat; Unternehmen Rohm & Haas/Dow Chemical), Acusol 445N (Polycarboxylat Natriumsalz;
Unternehmen Rohm & Haas/Dow Chemical), Acusol 590 (Polyarcrylat-copolymer; Unternehmen Rohm & Haas/Dow Chemical), Acusol 916 (Polyarcrylat Natriumsalz; Unternehmen Rohm & Haas/Dow Chemical), Sokalan CP42 (modifiziertes Polycarboxylat Natriumsalz; Unternehmen BASF), Sokalan PA 30CL (Polycarboxylat Natriumsalz; Unternehmen BASF), Dequest P 9000 (Polymaleinsäure; Unternehmen Thermphos), Alginsäure, Poly-2-acrylamido-2-methyl-1-propan-sulfonsäure, Poly-4-styrol sulfonsäure -co-maleinsäure Natriumsalz, Poly-acrylamido-co-acrylsäure Natriumsalz, Poly-methacrylsäure Natriumsalz, Poly-methyl vinyl ether-alt maleinsäure oder Polyvinylsulfonsäure Natriumsalz.

Bei den unter ii. angegebenen Substanzen handelt es sich kationische oder polykationische Substanzen, d.h. diese Substanzen tragen mindestens eine und bevorzugt mehrere positive Ladungen. Bevorzugt handelt es sich um ein Polymer mit mindestens einem positiv gelandenen Monomer, bevorzugt mit mehreren positiv geladenen Monomeren. Erfindungsgemäß bevorzugt ist dieses Polymer daher ein positiv geladenes Polymer. Beispiele für diesbezüglich bevorzugte Verbindungen sind Salze der Polyamine, Polyethyleneimine bzw. deren Copolymere, Salze der Polyallylamine, Salze der Polydiallyldimethylammonium-verbindungen oder Poly(acrylamide-co-diallyldimethylammonium-verbindungen.

Bei den unter iii. angegebenen Substanzen handelt es sich um Substanzen, die mindestens eine Hydroxyl- und/oder Polyhydroxyl-Gruppe und bevorzugt mehrere Hydroxyl- und/oder Polyhydroxyl-Gruppen aufweisen. Bevorzugt sind diesbezüglich beispielsweise Polyvinylalkohole, beispielsweise solche, die unter dem Handelsnamen Mowiol verfügbar sind (Unternehmen Kremer Pigmente GmbH & Co. KG).

Es wird an dieser Stelle ausdrücklich darauf hingewiesen, dass eine konkrete Substanz zu einer oder mehreren der vorstehend genannten Gruppen i. bis iii. zugehörig sein kann. Beispielsweise kann es sich um ein anionisches Polymer handeln, welches eine oder mehrere Hydroxyl- und/oder Polyhydroxyl-Gruppe(n) aufweist. Eine solche Substanz ist dann zugehörig zu den Gruppen i. und iii. Ebenso ist ein kationisches Polymer, welches eine oder mehrere Hydroxyl- und/oder Polyhydroxyl-Gruppe(n) aufweist, zugehörig zu den Gruppen ii. und iii.

Im Rahmen der vorliegenden Erfindung ebenfalls einsetzbar sind Derivate der vorstehend als zugehörig zu i., ii. oder iii. genannten Substanzen. Unter einem Derivat wird im Sinne der vorliegenden Anmeldung eine solche Substanz verstanden, die ausgehend von einer der vorstehend genannten Substanzen chemisch modifiziert ist, beispielsweise durch die Umwandlung einer Seitenkette oder durch kovalente Bindung einer anderen Verbindung an die Substanz. Bei solch einer Verbindung kann es sich beispielsweise um niedrigmolekulare Verbindungen wie Lipide oder Mono-, Oligo- oder Polysaccharide oder Amine bzw. Aminverbindungen handeln. Ferner kann die Substanz glykosyliert, hydrolysiert, oxidiert, N-methyliert, N-formyliert, N-acetyliert sein oder Methyl, Formyl, Ethyl, Acetyl, t-Butyl, Anisyl, Benzyl, Trifluroacetyl, N-hydroxysuccinimide, t-Butyloxycarbonyl, Benzoyl, 4-Methylbenzyl, Thioanizyl, Thiocresyl, Benzyloxymethyl, 4-Nitrophenyl, Benzyloxycarbonyl, 2-Nitrobenzoyl, 2-Nitrophenylsulphenyl, 4-Toluenesulphonyl, Pentafluorophenyl, Diphenylmethyl, 2-Chlorobenzyloxycarbonyl, 2,4,5-trichlorophenyl, 2-bromobenzyloxycarbonyl, 9-Fluorenylmethyloxycarbonyl, Tripheylmethyl, 2,2,5,7,8-pentamethyl-chroman-6-sulphonyl enthalten. Ebenso ist unter einem Derivat die kovalente oder nichtkovalente Bindung der Substanz an einen makromolekularen Träger zu verstehen, genauso wie auch ein nichtkovalenter Einschluss in geeignete makromolekulare Käfigstrukturen. Auch Kopplungen mit sonstigen makromolekularen Verbindungen, wie etwa Polyethylenglykol, können vorgenommen sein. Weitere bevorzugte chemische Modifikationen sind die Modifikation von einer oder mehreren der chemischen Gruppen -COOH, -OH, =NH, -NH₂ -SH zu -COOR, -OR, -NHR, -NR2, -NHR, -NR, -SR; wobei:
R ist-CH=CH-R2, -C=C-R2, -C(R2)=CH₂, -C(R2)=C(R3), -CH=NR2, -C(R2)=N-R3, ein 4-7 C-Ring System mit oder ohne Substitution, ein 4-7 Stickstoffheterozyklus mit oder ohne Substitution, oder eine C₂ bis C₈ Kette mit 1 bis 5 Doppel- oder Dreifachbindungen mit Substitutionen ausgewählt aus R1, R2, oder R3, wobei
-R1 ist H, -R, -NO₂, -CN, Halogenidsubstituent, -N₃, -C1-8 alkyl, -(CH₂)nCO₂R2, -C2-8 alkenyl-CO₂R2 -O(CH₂)nCO₂R2, -C(O)NR2R3, -P(O)(OR2)₂, alkyl substituiertes tetrazol-5-yl, -(CH₂)nO(CH₂)n aryl, -NR2R3, -(CH₂)n OR2, -(CH₂)n SR2, -N(R2)C(O)R3, -S(O₂)NR2R3, -N(R2)S(O₂)R3, -(CHR2)n NR2R3, -C(O)R3, (CH₂)n N(R3)C(O)R3, -N(R2)CR2R3, substituiertes oder nicht substituiertes (CH₂)n-zykloalkyl, substituiertes oder nicht substituiertes (CH₂)n-phenyl, oder -zyklus; wobei n eine Zahl größer als 1 ist;
-R2 ist H, Halogenidsubstituent, -alkyl, -halogenalkyl, -(CH₂)n-phenyl, -(CH₂)1-3-biphenyl, -(CH₂)1-4-Ph-N(SO₂-C1-2-alkyl)₂, -CO(CHR1)n-OR1, -(CHR1)n-Heterozyklus, -(CHR1)n-NH-CO-R1, -(CHR1)n-NH-SO₂R1, -(CHR1)n-Ph-N(SO₂-C1-2-alkyl)₂, -(CHR1)n-C(O)(CHR1)-NHR1, -(CHR1)n-C(S)(CHR1)-NHR1, -(CH₂)nO(CH₂)nCH₃, -CF₃, -C₂-C₅ acyl, -(CHR1)nOH, -(CHR1)nCO₂R1, -(CHR1)n-O-alkyl, -(CHR1)n-O-(CH₂)n-O-alkyl, -(CHR1)n-S-alkyl, -(CHR1)n-S(O)-alkyl, -(CHR1)n-S(O₂)-alkyl, -(CHR1)n-S(O₂)-NHR3, -(CHR3)n-N₃, -(CHR3)nNHR4, eine C₂ bis C₈ Kette Alken-Kette mit 1 bis 5 Doppelbindungen, eine C₂ bis C₈ Kette Alkin-Kette mit 1 bis 5 Dreifachbindungen, substituierter oder nicht substituierter -(CHR3)n Heterozyklus, substituiertes oder nicht substituiertes gesättigtes oder nicht gesättigtes -(CHR3)n Zykloalkyl; wobei n eine Zahl größer als 1 ist und R1 und R3 gleich oder unterschiedlich sein können;
-R3 ist H, -OH, -CN, substituiertes Alkyl, - C₂ bis C₈ Alkenyl, substituiertes oder nicht substituiertes Zykloalkyl, -N(R1)R2, gesättigter oder nicht gesättigter C₅ bis C₇ Heterozyklus oder Heterobizyklus von 4 bis 7 C-Atomen, -NR1, -NR2, -NR1R2 bestehend aus einem gesättigten oder nicht gesättigten Heterozyklus oder einem Heterobizyklus von 4 bis 7 C-Atomen;
-R4 ist H, -(CH₂)nOH, -C(O)OR5, -C(O)SR5, -(CH₂)n C(O)NR6R7, -O-C(O)-O-R6, eine Aminosäure oder ein Peptid; wobei n eine Zahl zwischen 0 und 4 ist;
-R5 ist H,
-R6 ist -C(R7)-(CH₂)n-O-C(O)-R8, -(CH₂)n-C(R7)-O-C(O)R8, -(CH₂)n-C(R7)-O-C(O)-O-R8, oder -C(R7)(CH₂)n-O-C(O)-O-R8; wobei n eine Zahl zwischen 0 und 4 ist; und
-R7 und R8 sind jeweils H, Alkyl, substituiertes Alkyl, Aryl, substituiertes Aryl, Alkenyl, substituiertes Alkenyl, Alkinyl, substituiertes Alkinyl, Heterozyklus, substituierter Heterozyklus, Alkylaryl, substituiertes Alkylaryl, Zykloalkyl, substituiertes Zykloalkyl, oder CH₂CO₂alkyl, wobei R7 und R8 gleich oder unterschiedlich sein können.

Erfindungsgemäß ist es weiter möglich, alle möglichen Kombinationen der vorstehend als zugehörig zu i., ii. oder iii. genannten Substanzen und/oder deren Derivate einzusetzen.

Ein erfindungsgemäßes flüssiges Wasch- oder Reinigungsmittel kann als solches oder nach Verdünnen mit Wasser zur Reinigung von Textilien und/oder harten Oberflächen eingesetzt werden. Eine solche Verdünnung kann leicht hergestellt werden, indem eine abgemessene Menge des Mittels in einer weiteren Menge Wasser verdünnt wird in bestimmten Gewichtsverhältnissen von Mittel : Wasser und optional diese Verdünnung geschüttelt wird, um eine gleichmäßige Verteilung des Mittels im Wasser sicherzustellen. Mögliche Gewichts- oder Volumenverhältnisse der Verdünnungen sind von 1:0 Mittel : Wasser bis 1:10000 oder 1:20000 Mittel : Wasser, vorzugsweise von 1:10 bis 1:2000 Mittel : Wasser.

Als flüssige Wasch- oder Reinigungsmittel können hierbei alle flüssigen bzw. fließfähigen Darreichungsformen dienen. "Fließfähig" im Sinne der vorliegenden Anmeldung sind dabei Mittel, welche gießbar sind und Viskositäten bis hin zu mehreren 10.000 mPas aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 5 bis 10000 mPas. Bevorzugte Mittel haben Viskositäten von 10 bis 8000 mPas, wobei Werte zwischen 120 bis 3000 mPas besonders bevorzugt sind. Ein flüssiges Wasch- oder Reinigungsmittel im Rahmen der vorliegenden Erfindung kann daher auch gelförmig oder pastenförmig sein, es kann als homogene Lösung oder Suspension vorliegen, sowie beispielsweise versprühbar oder in sonstigen üblichen Darreichungsformen konfektioniert sein. Zu den Waschmitteln zählen alle denkbaren Waschmittelarten, insbesondere Waschmittel für Textilien, Teppiche oder Naturfasern. Sie können für die manuelle und/oder auch die maschinelle Anwendung vorgesehen sein. Zu den Waschmitteln zählen ferner Waschhilfsmittel, die bei der manuellen oder maschinellen Textilwäsche zum eigentlichen Waschmittel hinzudosiert werden, um eine weitere Wirkung zu erzielen. Zu den Reinigungsmitteln werden alle, ebenfalls in sämtlichen genannten Darreichungsformen vorkommenden Mittel zur Reinigung und/oder Desinfektion harter Oberflächen, manuelle und maschinelle Geschirrspülmittel, Teppichreiniger, Scheuermittel, Glasreiniger, WC-Duftspüler, usw. gezählt. Textilvor- und Nachbehandlungsmittel sind schließlich auf der einen Seite solche Mittel, mit denen das Wäschestück vor der eigentlichen Wäsche in Kontakt gebracht wird, beispielsweise zum Anlösen hartnäckiger Verschmutzungen, auf der anderen Seite solche, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Desinfektionsmittel sind beispielsweise Händedesinfektionsmittel, Flächendesinfektionsmittel und Instrumentendesinfektionsmittel, die ebenfalls in den genannten Darreichungsformen vorkommen können.

In einer weiteren bevorzugten Ausführungsform der Erfindung ist das Wasch- oder Reinigungsmittel dadurch gekennzeichnet, dass es mindestens einen weiteren Inhaltsstoff umfasst, insbesondere einen, der ausgewählt ist aus der Gruppe bestehend aus Phosphonat, Tensid, Gerüststoff (Builder), nichtwässriges Lösungsmittel, Säure, wasserlösliches Salz, Verdickungsmittel sowie Kombinationen hiervon.

Phosphonate sind Salze und organische Verbindungen, insbesondere Ester, der Phosphonsäure. Als Salze existieren primäre (M'H₂PO₃ bzw. HP(O)(OH)(OM')) und sekundäre (M'₂HPO₃ bzw. HP(O)(OM')₂) Phosphonate, wobei M' für ein einwertiges Metall steht. Diese anorganischen Phosphonate und werden auch als primäre bzw. sekundäre Phosphite bezeichnet. Anorganische Phosphonate entstehen beispielsweise durch Umsetzung von Phosphonsäure HP(O)(OH)₂, insbesondere der stabilen tautomeren Form der Phosphorigsäure mit einem (primäre) oder zwei (sekundäre) Äquivalenten Base, beispielsweise Alkalimetallhydroxid. Im Rahmen der vorliegenden Erfindung bevorzugt sind organisch P-substituierte Phosphonate, die eine Phosphor-Kohlenstoff-Bindung aufweisen (Phosphor-organische Verbindungen). Ihre allgemeine Struktur ist R1P(O)(OR2)₂, mit R1 und/oder R2 = Alkyl, Aryl oder H, wobei die Alkyl- bzw. Arylreste weitere Substitutionen aufweisen oder weitere chemische Gruppen tragen können. Organisch P-substituierte Phosphonate entstehen beispielsweise durch Michaelis-Arbusov-Reaktion. Viele dieser Phosphonate sind löslich in Wasser. Einige technisch wichtige Phosphonate tragen ferner Amino-Gruppe(n) in der Art NR-(CH2)ₓ-PO(OH)₂ (R = Alkyl, Aryl oder H). Einige diese Aminophosphonate haben strukturelle Ähnlichkeiten mit Komplexbildnern wie EDTA, NTA oder DTPA und haben eine ähnliche Funktion. Zu besonders bevorzugten Phosphonaten im Rahmen der vorliegenden Erfindung zählen insbesondere Organophosphonate wie beispielsweise 1-Hydroxyethan-1,1-diphosphonsäure (HEDP), Aminotri(methylenphosphonsäure) (ATMP, auch bezeichnet als Amino-tris(methylenphosphonsäure) bzw. Nitrolotris(methylenphosphonsäure) (NTMP)), Diethylentriamin-penta(methylenphosphonsäure) (DTPMP oder DETPMP oder DTPNT), Ethylendiamintetra(methylenphosphonsäure) (EDTMP, auch bezeichnet als Ethylendiamin-tetra(methylenphosphonsäure) sowie 2-Phosphonobutan-1,2,4-tricarbonsäure (PBS-AM, auch bezeichnet als 2-Phosphonobutan-1,2,4-tricarbonsäure bzw. 3-Carboxy-3-phosphonoadipinsäure), die zumeist in Form ihrer Ammonium- oder Alkalimetallsalze eingesetzt werden. Besonders bevorzugt ist Diethylentriamin-penta(methylenphosphonsäure)-Natrium. Ein solches Phosphonat ist beispielsweise unter dem Handelsnamen Dequest® 2066 (Firma Thermphos) erhältlich.

Vorzugsweise ist das Phosphonat in einer Menge von 0,01 bis 2,5 Gew.-% und zunehmend bevorzugt von 0,02 bis 2 Gew.-%, von 0,03 bis 1,5 Gew.-% und insbesondere von 0,05 bis 1 Gew.-% in dem Wasch- oder Reinigungsmittel enthalten.

Als Tensid(e) können anionische, nichtionische, zwitterionische und/oder amphotere Tenside eingesetzt werden. Bevorzugt sind aus anwendungstechnischer Sicht Mischungen aus anionischen und nichtionischen Tensiden. Der Gesamttensidgehalt des flüssigen Wasch- oder Reinigungsmittels liegt vorzugsweise unterhalb von 60 Gew.-% und besonders bevorzugt unterhalb von 45 Gew.-%, bezogen auf das gesamte flüssige Wasch- oder Reinigungsmittel.

Geeignete nichtionische Tenside umfassen alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Insbesondere bevorzugt enthält das Wasch-, Reinigungs-, Nachbehandlungs- oder Waschhilfsmittel einen C₁₂₋₁₈-Fettalkohol mit 7 EO oder einen C₁₃₋₁₅-Oxoalkohol mit 7 EO als nichtionisches Tensid.

Der Gehalt an nichtionischen Tensiden beträgt in dem Wasch- oder Reinigungsmittel bevorzugt 3 bis 40 Gew.-%, vorzugsweise 5 bis 30 Gew.-% und insbesondere 7 bis 20 Gew.-%, jeweils bezogen auf das gesamte Wasch- oder Reinigungsmittel.

Neben den nichtionischen Tensiden kann das Wasch- oder Reinigungsmittel auch anionische Tenside enthalten. Als anionisches Tensid werden vorzugsweise Sulfonate, Sulfate, Seifen, Alkylphosphate, anionische Silikontenside und Mischungen daraus eingesetzt.

Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂₋₁₈-Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der C₁₂₋C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete anionische Tenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Auch bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Magnesium- oder Ammoniumsalze vorliegen. Vorzugsweise liegen die anionischen Tenside in Form ihrer Natriumsalze vor. Weitere bevorzugte Gegenionen für die anionischen Tenside sind auch die protonierten Formen von Cholin, Triethylamin oder Methylethylamin.

Der Gehalt eines Wasch- oder Reinigungsmittels an anionischen Tensiden kann 1 bis 40 Gew.-%, vorzugsweise 5 bis 30 Gew.-% und ganz besonders bevorzugt 10 bis 25 Gew.-%, jeweils bezogen auf das gesamte Wasch- oder Reinigungsmittel, betragen.

Als Gerüststoffe (Builder), die in dem Wasch- oder Reinigungsmittel enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Organische Gerüststoffe, welche in dem Wasch- oder Reinigungsmittel vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Bevorzugt werden allerdings lösliche Gerüststoffe, wie beispielsweise Citronensäure, oder Acrylpolymere mit einer Molmassen von 1.000 bis 5.000 g / mol bevorzugt in den flüssigen Wasch- oder Reinigungsmitteln eingesetzt.

Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt.

Die erfindungsgemäßen Wasch- oder Reinigungsmittel sind flüssig und enthalten vorzugsweise Wasser als Hauptlösungsmittel. Daneben können dem Wasch- oder Reinigungsmittel nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Di-isopropylenglykolmonomethylether, Diisopropylenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel. Es ist allerdings bevorzugt, dass das Wasch- oder Reinigungsmittel ein Polyol als nicht-wässriges Lösungsmittel enthält. Das Polyol kann insbesondere Glycerin, 1,2-Propandiol, 1,3-Propandiol, Ethylenglycol, Diethylenglycol und/oder Dipropylenglycol umfassen. Insbesondere bevorzugt enthält das Wasch- oder Reinigungsmittel eine Mischung aus einem Polyol und einem einwertigen Alkohol. Nichtwässrige Lösungsmittel können in dem Wasch- oder Reinigungsmittel in Mengen zwischen 0,5 und 15 Gew.-%, bevorzugt aber unter 12 Gew.-% und eingesetzt werden.

Zur Einstellung eines gewünschten, sich durch die Mischung der übrigen Komponenten nicht von selbst ergebenden pH-Werts können die Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Mitteln in Mengen von vorzugsweise nicht über 20 Gew.-%, insbesondere von 1,2 Gew.-% bis 17 Gew.-%, enthalten.

Ein Mittel im Sinne der Erfindung kann weiterhin ein oder mehrere wasserlösliche Salze enthalten, die beispielsweise zur Viskositätseinstellung dienen. Es kann sich dabei um anorganische und/oder organische Salze handeln. Einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben. Einsetzbare organische Salze sind beispielsweise farblose wasserlösliche Alkalimetall-, Erdalkalimetall-, Ammonium-, Aluminium- und/oder Übergangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Zur Verdickung kann ein erfindungsgemäßes Mittel ein oder mehrere Verdickungsmittel enthalten. Bevorzugt ist das Verdickungsmittel ausgewählt aus der Gruppe umfassend Xanthan, Guar, Carrageenan, Agar-Agar, Gellan, Pektin, Johannisbrotkernmehl und Mischungen daraus. Diese Verbindungen sind auch in Gegenwart von anorganischen Salzen effektive Verdickungsmittel. In einer besonders bevorzugten Ausführungsform enthält das Wasch- oder Reinigungsmittel Xanthan als Verdickungsmittel, da Xanthan auch in Gegenwart von hohen Salzkonzentrationen effektiv verdickt und eine makroskopische Auftrennung der kontinuierlichen Phase verhindert. Zusätzlich stabilisiert das Verdickungsmittel die kontinuierliche, tensidarme Phase und verhindert eine makroskopische Phasenseparation.

Alternativ oder ergänzend können auch (Meth)Acrylsäure(co)polymere als Verdickungsmittel eingesetzt werden. Geeignete Acryl- und Methacryl(co)polymere umfassen beispielsweise die hochmolekularen mit einem Polyalkenylpolyether, insbesondere einem Allylether von Saccharose,

Pentaerythrit oder Propylen, vernetzten Homopolymere der Acrylsäure (INCI-Bezeichnung gemäß "International Dictionary of Cosmetic Ingredients" der "The Cosmetic, Toiletry and Fragrance Association (CTFA)": Carbomer), die auch als Carboxyvinylpolymere bezeichnet werden. Solche Polyacrylsäuren sind unter anderem unter den Handelsnamen Polygel® und Carbopol® erhältlich. Weiterhin sind beispielsweise folgende Acrylsäure-Copolymere geeignet: (i) Copolymere von zwei oder mehr Monomeren aus der Gruppe der Acrylsäure, Methacrylsäure und ihrer einfachen, vorzugsweise mit C₁₋₄-Alkanolen gebildeten, Ester (INCI Acrylates Copolymer), die beispielsweise unter den Handelsnamen Aculyn®, Acusol® oder Tego® Polymer erhältlich sind; (ii) vernetzte hochmolekulare Acrylsäure-Copolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₋₄-Alkanolen gebildeten, Ester (INCI Acrylates/C₁₀₋₃₀ Alkyl Acrylate Crosspolymer) gehören und die beispielsweise unter dem Handelsnamen Carbopol® erhältlich sind. Weitere geeignete Polymere sind (Meth)Acrylsäure(co)polymere des Typs Sokalan®.

Es kann bevorzugt sein, dass das erfindungsgemäße Wasch- oder Reinigungsmittel ein (Meth)Acrylsäure(co)polymer in Kombination mit einem weiteren Verdickungsmittel, vorzugsweise Xanthan, enthält. Das Wasch- oder Reinigungsmittel kann 0,05 bis 1,5 Gew.-% und vorzugsweise 0,1 bis 1 Gew.-%, jeweils bezogen auf das gesamte Wasch- oder Reinigungsmittel, Verdickungsmittel enthalten. Die Menge an eingesetztem Verdickungsmittel ist dabei abhängig von der Art des Verdickungsmittels und dem gewünschten Grad der Verdickung.

Flüssige beziehungsweise pastöse erfindungsgemäße Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

Erfindungsgemäße Wasch- oder Reinigungsmittel können ausschließlich eine Protease und eine Cellulase enthalten wie beschrieben. Alternativ können sie auch weitere hydrolytische Enzyme oder andere Enzyme in einer für die Wirksamkeit des Mittels zweckmäßigen Konzentration enthalten. Einen weiteren Gegenstand der Erfindung stellen somit Mittel dar, die ferner eines oder mehrere weitere Enzyme umfassen, wobei prinzipiell alle im Stand der Technik für diese Zwecke etablierten Enzyme einsetzbar sind. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase, sowie deren Gemische. Weitere Enzyme sind in dem Mittel vorteilhafterweise jeweils in einer Gesamtmenge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solches Synergismus vor zwischen der erfindungsgemäß enthaltenen Protease und einem weiteren Enzym eines erfindungsgemäßen Mittels, darunter insbesondere zwischen der genannten Protease und der Cellulase und/oder einer Lipase und/oder einer Mannanase und/oder einer Amylase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Einen weiteren Erfindungsgegenstand stellt die Verwendung eines erfindungsgemäßen Wasch- oder Reinigungsmittels zur Entfernung von Anschmutzungen, insbesondere von protease-und/oder cellulase-sensitiven Anschmutzungen, auf Textilien oder harten Oberflächen, d.h. zur Reinigung von Textilien oder von harten Oberflächen, dar. Denn erfindungsgemäße Mittel können, insbesondere auf Grund der enthaltenen Kombination von Protease und Cellulase, vorteilhaft dazu verwendet werden, um von Textilien oder von harten Oberflächen entsprechende Verunreinigungen zu beseitigen. Ausführungsformen dieses Erfindungsgegenstandes stellen beispielsweise die Handwäsche, die manuelle Entfernung von Flecken von Textilien oder von harten Oberflächen oder die Verwendung im Zusammenhang mit einem maschinellen Verfahren dar. Alle Sachverhalte, Gegenstände und Ausführungsformen, die für erfindungsgemäße Wasch- oder Reinigungsmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

Einen weiteren Erfindungsgegenstand stellt ein Verfahren zur Reinigung von Textilien oder von harten Oberflächen dar, wobei in mindestens einem Verfahrensschritt ein erfindungsgemäßes Wasch- oder Reinigungsmittel angewendet wird.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung bzw. Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels bereichert werden und stellen dann Ausführungsformen der vorliegenden Erfindung dar. Alle Sachverhalte, Gegenstände und Ausführungsformen, die für erfindungsgemäße Wasch- oder Reinigungsmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

In einer bevorzugten Ausführungsform ist das Verfahren dadurch gekennzeichnet, dass die Cellulase in der Waschflotte in einer Konzentration von 0,0000004 bis 0,0006 Gew.-%, bevorzugt von 0,0000008 bis 0,00048 Gew.-% vorliegt, und/oder dass die Protease in der Waschflotte in einer Konzentration von 0,00009 bis 0,0005 Gew.-%, bevorzugt von 0,00015 bis 0,00035 Gew.-% vorliegt, wobei die Angaben auf Aktivprotein in der Waschflotte bezogen sind. In einer weiteren bevorzugten Ausführungsform ist das Verfahren dadurch gekennzeichnet, dass es bei einer Temperatur zwischen 10°C und 50°C, bevorzugt zwischen 10°C und 40°C und besonders bevorzugt zwischen 20°C und 40°C durchgeführt wird.

In erfindungsgemäßen Mitteln eingesetzte Proteasen sind entsprechend der vorstehenden Ausführungen vorteilhaft in erfindungsgemäßen Wasch- und Reinigungsmitteln sowie Verfahren, insbesondere Wasch- und Reinigungsverfahren, einsetzbar. Sie können also vorteilhaft dazu verwendet werden, um in entsprechenden Mitteln eine proteolytische Aktivität bereitzustellen.

Weiterhin offenbart hierin ist die Verwendung einer Protease,
(a1) die eine Aminosäuresequenz umfasst, die zu der in SEQ ID NO. 1 angegebenen
   Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, oder
(a2) die eine Aminosäuresequenz umfasst, die zu der in SEQ ID NO. 1 angegebenen
   Aminosäuresequenz zu mindestens 80% identisch ist und die an Position 99 in der Zählung gemäß SEQ ID NO. 1 die Aminosäure Asparagin (N) oder Glutamin (Q) aufweist, oder
(a3) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Alanin (A) oder Glycin (G) oder Serin (S) aufweist,
   zur Bereitstellung einer proteolytischen Aktivität in einem flüssigen Wasch- oder Reinigungsmittel, welches ferner eine Cellulase umfasst.

In einer weiteren Ausführungsform ist diese Verwendung dadurch gekennzeichnet, dass die Protease ferner in der Zählung gemäß SEQ ID NO. 1 mindestens eine der folgenden Aminosäuren aufweist:
(a) Threonin an Position 3 (3T),
(b) Isoleucin an Position 4 (41),
(c) Alanin, Threonin oder Arginin an Position 61 (61A, 61T oder 61 R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (1991),
(h) Asparaginsäure, Glutaminsäure oder Glycin an Position 211 (211 D, 211 E oder 211 G),
(i) Kombinationen der Aminosäuren (a) bis (h).

Einen weiteren Gegenstand der Erfindung bildet die Verwendung einer Protease, die ausgewählt ist aus der Gruppe bestehend aus
a. Protease umfassend eine Aminosäuresequenz gemäß SEQ ID NO. 2;
b. Protease, die eine gegenüber SEQ ID NO. 2 in mindestens einer Position veränderte Aminosäuresequenz umfasst, wobei die Veränderung in der Zählung gemäß SEQ ID NO. 1 ausgewählt ist aus der Gruppe bestehend aus:
   i. Threonin an Position 3 (3T),
   ii. Isoleucin an Position 4 (41),
   iii. Alanin, Threonin oder Arginin an Position 61 (61A, 61T oder 61 R),
   iv. Asparaginsäure oder Glutaminsäure an Position 154 (154D oder 154E),
   v. Prolin an Position 188 (188P),
   vi. Methionin an Position 193 (193M),
   vii. Isoleucin an Position 199 (1991),
   viii. Asparaginsäure, Glutaminsäure oder Glycin an Position 211 (211 D, 211 E oder 211 G),
   ix. Kombinationen der Aminosäuren (i) bis (viii);
zur Bereitstellung einer proteolytischen Aktivität in einem flüssigen Wasch- oder Reinigungsmittel, welches ferner eine Cellulase umfasst.

Weiterhin offenbart hierin ist die Verwendung einer Protease, die ausgewählt ist aus der Gruppe bestehend aus
a. Protease umfassend eine Aminosäuresequenz gemäß SEQ ID NO. 3 oder SEQ ID NO. 4 oder SEQ ID NO. 5 oder SEQ ID NO. 6 oder SEQ ID NO. 7 oder SEQ ID NO. 8;
b. Protease, die eine gegenüber SEQ ID NO. 3 oder SEQ ID NO. 4 oder SEQ ID NO. 5 oder SEQ ID NO. 6 oder SEQ ID NO. 7 oder SEQ ID NO. 8 in mindestens einer Position veränderte Aminosäuresequenz umfasst, wobei die Veränderung in der Zählung gemäß SEQ ID NO. 1 ausgewählt ist aus der Gruppe bestehend aus:
   x. Threonin an Position 3 (3T),
   xi. Isoleucin an Position 4 (41),
   xii. Alanin, Threonin oder Arginin an Position 61 (61A, 61T oder 61R),
   xiii. Asparaginsäure oder Glutaminsäure an Position 154 (154D oder 154E),
   xiv. Prolin an Position 188 (188P),
   xv. Methionin an Position 193 (193M),
   xvi. Isoleucin an Position 199 (1991),
   xvii. Asparaginsäure, Glutaminsäure oder Glycin an Position 211 (211 D, 211 E oder 211G),
   xviii. Kombinationen der Aminosäuren (i) bis (viii);
zur Bereitstellung einer proteolytischen Aktivität in einem flüssigen Wasch- oder Reinigungsmittel, welches ferner eine Cellulase umfasst.

Alle Sachverhalte, Gegenstände und Ausführungsformen, die für erfindungsgemäße Wasch- oder Reinigungsmittel beschrieben sind, sind auch auf die genannten Verwendungen anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verwendungen gilt.

Beispiel: Ermittlung der Lagerstabilität erfindungsgemäßer flüssiger Waschmittel

Als Waschmittel-Basisrezepturen dienten
a) ein erstes Flüssigwaschmittel folgender Zusammensetzung (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettaikohoiethersuifat), 24-28% nichtionische Tenside, 1% Borsäure, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001 % Farbstoff, Rest demineralisiertes Wasser. Als Cellulase enthalten war 0,15 Gew.-% Ecostone N400® (Cellulasepräparation der Firma AB Enzymes).
b) ein zweites Flüssigwaschmittel folgender Zusammensetzung:

| Inhaltsstoff | Gew.-% |
|---|---|
| C₁₂₋₁₈-Fettalkohol mit 7 EO | 7,5 |
| Lin. C₁₀-C₁₃-Alkylbenzolsulfonat (Na-Salz) | 8,5 |
| Kokosfettsäure (Na-Salz) | 14,6 |
| Laurylethersulfat mit 2 EO (Na-Salz) | 13,0 |
| Citronensäure (Na-Salz) | 3,1 |
| Borsäure (Na-Salz) | 1,0 |
| Polyacrylat-Verdicker | 0,4 |
| Propylenglykol | 2,1 |
| Silikon-Entschäumer | 0,2 |
| Cellulasepräparation Ecostone N400® (Firma AB Enzymes) | 0,1 |
| Wasser | Ad 100 |

Die Waschmittel-Basis-Rezepturen wurden für die verschiedenen Versuchsansätze mit folgenden Proteasen versetzt, wobei die Angaben auf aktives Protein bezogen sind:
Ansatz 1: Leistungsverbesserte Variante F49 der Protease aus Bacillus lentus gemäß WO 95/23221 (Arg an Position 99 (99R)): 0,4 mg/ml (0,04 Gew.-%) in den Flüssigwaschmitteln gemäß a) und b).
Ansatz 2: Protease, die in Figur 2 bzw. SEQ ID NO. 3 der internationalen Offenlegungsschrift WO 03/057713 offenbart ist (Ser an Position 99 (99S; Identität zu SEQ ID NO. 1 < 80%): 0,4 mg/ml (0,04 Gew.-%) in dem Flüssigwaschmittel gemäß a), 0,3 mg/ml (0,03 Gew.-%) in dem Flüssigwaschmittel gemäß b).
Ansatz 3: Protease umfassend eine Aminosäuresequenz gemäß SEQ ID NO. 2 (Glu an Position 99 (99E)): 0,4 mg/ml (0,04 Gew.-%) in dem Flüssigwaschmittel gemäß a), 0,3 mg/ml (0,03 Gew.-%) in dem Flüssigwaschmittel gemäß b).

Die Waschmittel gemäß der jeweiligen Ansätze 1, 2 und 3 wurden hinsichtlich ihrer Lagerstabilität überprüft. Hierfür wurden die Waschmittel bei einer Temperatur von 30°C für den jeweils angegebenen Zeitraum gelagert und die jeweilige cellulolytische Restaktivität bestimmt. Unter definierten Reaktionsbedingungen (100 mM Natriumphosphatpuffer pH 7.5, 40°C, 15 min) wurden die zu untersuchenden Proben mit einem Substrat (1,25% Carboxymethylcellulose) inkubiert. Unter alkalischen Bedingungen reagierten diese mit p-Hydroxybenzoesäure-hydrazid (PAHBAH) unter Anwesenheit von Bismuth zu einem gelben Farbstoff, der photometrisch bei einer Wellenlänge von 410 nm bestimmt wurde. Die der Färbung entsprechende Menge des freigesetzten Zuckers ist dabei ein Maß für die Enzymaktivität (vgl. Lever, Anal. Biochem., 1972, 47 & 1977, 81). Die erhaltenen cellulolytischen Restaktivitäten sind in nachfolgender Tabelle 1 angegeben.

**Tabelle1:**

| Waschmittel gemäß | a) | | | b) | | |
|---|---|---|---|---|---|---|
| | Anfang | 4 Wochen | 8 Wochen | Anfang | 4 Wochen | 8 Wochen |
| Ansatz 1 | 100% | 42% | 27% | 100% | 33% | 21% |
| Ansatz 2 | 100% | 36% | 24% | 100% | 42% | 24% |
| Ansatz 3 | 100% | 46% | 32% | 100% | 49% | 26% |

Es wird deutlich, dass erfindungsgemäße Waschmittel eine deutlich verbesserte cellulolytische Restaktivität und damit Lagerstabilität aufweisen im Vergleich mit den Waschmitteln der Ansätze 1 und 2.

## Patentansprüche

1. Flüssiges Wasch- oder Reinigungsmittel umfassend
(a) eine Protease, die ausgewählt ist aus der Gruppe bestehend aus
a. Protease umfassend eine Aminosäuresequenz gemäß SEQ ID NO. 2;
b. Protease, die eine gegenüber SEQ ID NO. 2 in mindestens einer Position veränderte Aminosäuresequenz umfasst, wobei die Veränderung in der Zählung gemäß SEQ ID NO. 1 ausgewählt ist aus der Gruppe bestehend aus:
i. Threonin an Position 3 (3T),
ii. Isoleucin an Position 4 (41),
iii. Alanin, Threonin oderArginin an Position 61 (61A, 61T oder 61 R),
iv. Asparaginsäure oder Glutaminsäure an Position 154 (154D oder 154E),
v. Prolin an Position 188 (188P),
vi. Methionin an Position 193 (193M),
vii. Isoleucin an Position 199 (1991),
viii. Asparaginsäure, Glutaminsäure oder Glycin an Position 211 (211 D, 211 E oder 211 G),
ix. Kombinationen der Aminosäuren (i) bis (viii);
(b) eine Cellulase.

2. Wasch oder Reinigungsmittel nach Anspruch 1, **dadurch gekennzeichnet, dass** die Cellulase in einer Menge von 1 x 10⁻⁸ bis 5 Gewichts-Prozent, bezogen auf aktives Protein, enthalten ist, und/oder dass die Protease in einer Menge von 1 x 10⁻⁸ bis 5 Gewichts-Prozent, bezogen auf aktives Protein, enthalten ist.

3. Wasch- oder Reinigungsmittel nach einem der Ansprüche 1 oder 2, **dadurch gekennzeichnet, dass** es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

4. Wasch- oder Reinigungsmittel nach einem der Ansprüche 1 bis 3, **dadurch gekennzeichnet, dass** es mindestens einen weiteren Inhaltsstoff umfasst, insbesondere einen, der ausgewählt ist aus der Gruppe bestehend aus Phosphonat, Tensid, Gerüststoff (Builder), nichtwässriges Lösungsmittel, Säure, wasserlösliches Salz, Verdickungsmittel sowie Kombinationen hiervon.

5. Wasch- oder Reinigungsmittel nach einem der Ansprüche 1 bis 4, **dadurch gekennzeichnet, dass** es mindestens ein weiteres Enzym umfasst, insbesondere eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase, sowie deren Gemische.

6. Verwendung eines Wasch- oder Reinigungsmittels nach einem der Ansprüche 1 bis 5 zur Entfernung von protease-sensitiven Anschmutzungen auf Textilien oder harten Oberflächen.

7. Verfahren zur Reinigung von Textilien oder harten Oberflächen, **dadurch gekennzeichnet, dass** in mindestens einem Verfahrensschritt ein Wasch- oder Reinigungsmittel nach einem der Ansprüche 1 bis 5 angewendet wird.

8. Verfahren nach Anspruch 7, **dadurch gekennzeichnet, dass** die Cellulase in der Waschflotte in einer Konzentration von 0,0000004 bis 0,0006 Gew.-% vorliegt, und/oder dass die Protease in der Waschflotte in einer Konzentration von 0,00009 bis 0,0005 Gew.-% vorliegt.

9. Verfahren nach Anspruch 7 oder 8, **dadurch gekennzeichnet, dass** es bei einer Temperatur zwischen 10°C und 50°C, bevorzugt zwischen 10°C und 40°C und besonders bevorzugt zwischen 20°C und 40°C durchgeführt wird.

10. Verwendung einer Protease, die ausgewählt ist aus der Gruppe bestehend aus
a. Protease umfassend eine Aminosäuresequenz gemäß SEQ ID NO. 2;
b. Protease, die eine gegenüber SEQ ID NO. 2 in mindestens einer Position veränderte Aminosäuresequenz umfasst, wobei die Veränderung in der Zählung gemäß SEQ ID NO. 1 ausgewählt ist aus der Gruppe bestehend aus:
i. Threonin an Position 3 (3T),
ii. Isoleucin an Position 4 (41),
iii. Alanin, Threonin oderArginin an Position 61 (61A, 61T oder 61 R),
iv. Asparaginsäure oder Glutaminsäure an Position 154 (154D oder 154E),
v. Prolin an Position 188 (188P),
vi. Methionin an Position 193 (193M),
vii. Isoleucin an Position 199 (1991),
viii. Asparaginsäure, Glutaminsäure oder Glycin an Position 211 (211 D, 211 E oder 211G),
ix. Kombinationen der Aminosäuren (i) bis (viii);
zur Bereitstellung einer proteolytischen Aktivität in einem flüssigen Wasch- oder Reinigungsmittel, welches eine Cellulase umfasst.

## Claims

1. A liquid detergent or cleaner comprising
(a) a protease which is selected from the group consisting of
a. protease comprising an amino acid sequence according to SEQ ID No. 2;
b. protease which comprises an amino acid sequence modified in at least one position compared to SEQ ID No. 2, where the modification in the count according to SEQ ID No. 1 is selected from the group consisting of:
i. threonine at position 3 (3T),
ii. isoleucine at position 4 (41),
iii. alanine, threonine or arginine at position 61 (61A, 61T or 61R),
iv. aspartic acid or glutamic acid at position 154 (154D or 154E),
v. proline at position 188 (188P),
vi. methionine at position 193 (193M),
vii. isoleucine at position 199 (1991),
viii. aspartic acid, glutamic acid or glycine at position 211 (211D, 211E or 211G),
ix. combinations of amino acids (i) to (viii);
(b) a cellulase.

2. The detergent or cleaner according to claim 1, wherein the cellulase is present in an amount of 1 x 10⁻⁸ to 5 percent by weight, based on active protein, and/or the protease is present in an amount of 1 x 10⁻⁸ to 5 percent by weight, based on active protein.

3. The detergent or cleaner according to either one of claims 1 and 2, wherein it additionally comprises a component which is selected from
i. anionic and/or polyanionic substance, and/or
ii. cationic and/or polycationic substance, and/or
iii. substance having hydroxyl and/or polyhydroxyl group(s).

4. The detergent or cleaner according to any one of claims 1 to 3, wherein it comprises at least one further ingredient, in particular one which is selected from the group consisting of phosphonate, surfactant, builder, nonaqueous solvent, acid, watersoluble salt, thickener, and combinations thereof.

5. The detergent or cleaner according to any one of claims 1 to 4, wherein it comprises at least one further enzyme, in particular a protease, amylase, cellulase, hemicellulase, mannanase, tannase, xylanase, xanthanase, xyloglucanase, ß-glucosidase, pectinase, carrageenase, perhydrolase, oxidase, oxidoreductase or a lipase, and mixtures thereof.

6. The use of a detergent or cleaner according to any one of claims 1 to 5 for removing protease-sensitive soilings on textiles or hard surfaces.

7. A method of cleaning textiles or hard surfaces, wherein, in at least one method step, a detergent or cleaner according to any one of claims 1 to 5 is used.

8. The method according to claim 7, wherein the cellulase is present in the wash liquor in a concentration of from 0.0000004 to 0.0006% by weight, and/or the protease is present in the wash liquor in a concentration of from 0.00009 to 0.0005% by weight.

9. The method according to claim 7 or 8, wherein it is carried out at a temperature between 10°C and 50°C, preferably between 10°C and 40°C and particularly preferably between 20°C and 40°C.

10. The use of a protease which is selected from the group consisting of
a. protease comprising an amino acid sequence according to SEQ ID No. 2;
b. protease which comprises an amino acid sequence modified in at least one position compared to SEQ ID No. 2, where the modification in the count according to SEQ ID No. 1 is selected from the group consisting of:
i. threonine at position 3 (3T),
ii. isoleucine at position 4 (41),
iii. alanine, threonine or arginine at position 61 (61A, 61T or 61R),
iv. aspartic acid or glutamic acid at position 154 (154D or 154E),
v. proline at position 188 (188P),
vi. methionine at position 193 (193M),
vii. isoleucine at position 199 (1991),
viii. aspartic acid, glutamic acid or glycine at position 211 (211D, 211E or 211G),
ix. combinations of amino acids (i) to (viii);
for providing a proteolytic activity in a liquid detergent or cleaner which comprises a cellulase.

## Revendications

1. Détergent liquide, comprenant :
(a) une protéase, qui est choisie dans le groupe constitué par :
a. les protéases comprenant une séquence d'acides aminés selon SEQ ID NO. 2 ;
b. les protéases qui comprennent une séquence d'acides aminés modifiée en au moins une position par rapport à SEQ ID NO. 2, la modification dans la numérotation selon SEQ ID NO. 1 étant choisie dans le groupe constitué par :
i. la thréonine à la position 3 (3T),
ii. l'isoleucine à la position 4 (4I),
iii. l'alanine, la thréonine ou l'arginine à la position 61 (61A, 61T ou 61R),
iv. l'acide asparaginique ou l'acide glutamique à la position 154 (154D ou 154E),
v. la proline à la position 188 (188P),
vi. la méthionine à la position 193 (193M),
vii. l'isoleucine à la position 199 (199I),
viii. l'acide asparaginique, l'acide glutamique ou la glycine à la position 211 (211D, 211E ou 211G),
ix. des combinaisons des acides aminés (i) à (viii) ;
(b) une cellulase.

2. Détergent selon la revendication 1, **caractérisé en ce que** la cellulase est contenue en une quantité de 1 x 10⁻⁸ à 5 pour cent en poids, par rapport à la protéine active et/ou **en ce que** la protéase est contenue en un quantité de 1 x 10⁻⁸ à 5 pour cent en poids, par rapport à la protéine active.

3. Détergent selon l'une quelconque des revendications 1 ou 2, **caractérisé en ce qu'**il comprend en outre un composant, qui est choisi parmi :
i. une substance anionique et/ou polyanionique et/ou
ii. une substance cationique et/ou polycationique et/ou
iii. une substance comprenant (un ou plusiers) groupes hydroxyle et/ou polyhydroxyle.

4. Détergent selon l'une quelconque des revendications 1 à 3, **caractérisé en ce qu'**il comprend au moins un autre constituant, notamment un qui est choisi dans le groupe constitué par un phosphonate, un tensioactif, un matériau de squelette (adjuvant), un solvant non aqueux, un acide, un sel soluble dans l'eau, un épaississant et leurs combinaisons.

5. Détergent selon l'une quelconque des revendications 1 à 4, **caractérisé en ce qu'**il comprend au moins une enzyme supplémentaire, notamment une protéase, une amylase, une cellulase, une hémicellulase, une mannanase, une tannase, une xylanase, une xanthanase, une xyloglucanase, une β-glucosidase, une pectinase, une carragénase, une perhydrolase, une oxydase, une oxydoréductase ou une lipase, ainsi que leurs mélanges.

6. Utilisation d'un détergent selon l'une quelconque des revendications 1 à 5 pour l'élimination de salissures sensibles aux protéases sur des textiles ou des surfaces dures.

7. Procédé de nettoyage de textiles ou de surfaces dures, **caractérisé en ce qu'**un détergent selon l'une quelconque des revendications 1 à 5 est utilisé dans au moins une étape de procédé.

8. Procédé selon la revendication 7, **caractérisé en ce que** la cellulase est présente dans le bain de lavage en une concentration de 0,0000004 à 0,0006 % en poids, et/ou **en ce que** la protéase est présente dans le bain de lavage en une concentration de 0,00009 à 0,0005 % en poids.

9. Procédé selon la revendication 7 ou 8, **caractérisé en ce qu'**il est réalisé à une température comprise entre 10 °C et 50 °C, de préférence entre 10 °C et 40 °C, et de manière particulièrement préférée entre 20 °C et 40 °C.

10. Utilisation d'une protéase, qui est choisie dans le groupe constitué par
a. les protéases comprenant une séquence d'acides aminés selon SEQ ID NO. 2 ;
b. les protéases qui comprennent une séquence d'acides aminés modifiée en au moins une position par rapport à SEQ ID NO. 2, la modification dans la numérotation selon SEQ ID NO. 1 étant choisie dans le groupe constitué par :
i. la thréonine à la position 3 (3T),
ii. l'isoleucine à la position 4 (4I),
iii. l'alanine, la thréonine ou l'arginine à la position 61 (61A, 61T ou 61R),
iv. l'acide asparaginique ou l'acide glutamique à la position 154 (154D ou 154E),
v. la proline à la position 188 (188P),
vi. la méthionine à la position 193 (193M),
vii. l'isoleucine à la position 199 (199I),
viii. l'acide asparaginique, l'acide glutamique ou la glycine à la position 211 (211D, 211E ou 211G),
ix. des combinaisons des acides aminés (i) à (viii) ;
pour conférer une activité protéolytique à un détergent liquide qui comprend une cellulase.
